(19) Europäisches Patentamt
European Patent Office
Office européen des brevets

(11) Veröffentlichungsnummer: **0 061 530**
**A1**

(12) **EUROPÄISCHE PATENTANMELDUNG**

(21) Anmeldenummer: 81110022.1

(22) Anmeldetag: 01.12.81

(51) Int. Cl.³: A 23 C 9/12
C 12 P 19/14, C 12 N 11/08

(30) Priorität: 28.03.81 DE 3112336

(43) Veröffentlichungstag der Anmeldung:
06.10.82 Patentblatt 82/40

(84) Benannte Vertragsstaaten:
DE FR IT NL

(71) Anmelder: Röhm GmbH
Kirschenallee Postfach 4242
D-6100 Darmstadt 1(DE)

(72) Erfinder: Plainer, Hermann, Dr.
Am Wembach 15
D-6107 Reinheim 1(DE)

(72) Erfinder: Sprössler, Bruno, Dr.
Auf der Schmelz 93
D-6101 Rossdorf(DE)

(54) **Verfahren zur Spaltung von Milchzucker.**

(57) Milchzucker wird in neutraler Lactoselösung, z.B. Milch oder Süßmolke, mittels trägergebundener Pilzlactase, insbesondere aus Aspergillus oryzae, bei einer Temperatur über 50°C mit hohem Hydrolysegrad gespalten. Bakterienwachstum wird durch die hohe Temperatur vermieden. Die Aktivität des vorzugsweise covalent gebundenen Enzyms bleibt über mehrere Monate erhalten.

EP 0 061 530 A1

Croydon Printing Company Ltd.

Verfahren zur Spaltung von Milchzucker

Die Erfindung betrifft die Spaltung von Milchzucker in wäßriger Lösung. Technische Verfahren zur Milchzuckerspaltung sind dann besonders wirtschaftlich, wenn sie mit einem immobilisierten Enzym durchgeführt werden. Als Enzyme kommen Lactasen bzw. ß-Galactosidasen aus Bacterien, Hefen und Schimmelpilzen in Betracht.

In der DE-OS 28 39 737 wurde die Milchzuckerspaltung mit einer Lactase aus Escherichia coli, die adsorptiv an einen hydrophobisierten Träger gebunden ist, vorgeschlagen. Gegen den Einsatz dieses Enzyms im Lebensmittelgebiet bestehen Vorbehalte.

Hefelactasen werden in einem von M. Pastore (Latte 1977, 2/7-8, S. 456-60) beschriebenen Verfahren zur Lactosespaltung in Milch angewendet. Das Enzym ist in Celluloseacetatfasern immobilisiert. Um unerwünschtes Bacterienwachstum im Reaktor zu unterdrücken, wird die Hydrolyse bei einer Temperatur von 4°C durchgeführt, bei der sie ziemlich langsam verläuft. Beim Verfahren der DE-OS 21 51 534 wird die Milch nur kurzzeitig für die Hydrolyse auf 25°C erwärmt und anschließend sofort auf niedrige Temperaturen abgekühlt. Auch bei diesem Verfahren kann im Reaktor bei 25°C Bakterienwachstum auftreten, wodurch die Durchflußgeschwindigkeit herabgesetzt und die Milchqualität beeinträchtigt wird. Bei Temperaturen über 50°C tritt kein Bakterienwachstum mehr auf, jedoch vertragen die Hefelactasen diese Temperaturen nicht.

Pilzlactasen sind im Handel zu günstigen Preisen erhältlich, haben aber ein im sauren Bereich liegendes Wirkungsoptimum. Sie werden daher gemäß DE-OS 28 18 086 in trägergebundener Form zur Milchzuckerspaltung in saurer Lösung eingesetzt. Bei 40°C zeigt sich eine langdauernde Aktivität des trägergebundenen Enzyms. Diese Temperatur ist aber noch zu niedrig, um Bakterienwachstum auszuschließen. Versuche bei 55°C ergaben bei sonst gleichen Bedingungen nur noch eine Halbwertzeit von wenigen Tagen. Beim etwa neutralen pH-Wert der Milch ist die Aktivität der Pilzlactasen für ein technisches Verfahren zu gering, insbesondere, wenn zur Vermeidung von Bakterienwachstum bei tiefer Temperatur gearbeitet wird.

Es bestand die Aufgabe, die Spaltung von Milchzucker in etwa neutraler wäßriger Lösung mit einem handelsüblichen, technisch verfügbaren Enzym bei hoher Hydrolysegeschwindigkeit unter Bedingungen durchzuführen, bei denen Bakterienwachstum ausgeschlossen ist.

In der europäischen Patentanmeldung 6035 wird ein Verfahren beschrieben, bei dem dieses Ziel schon weitgehend erreicht wird. Man verwendet dazu eine trägergebundene Lactase aus einer Kultur eines bestimmten Bac. stearothermophilus-Stammes und führt die Hydrolyse bei 55 - 65°C durch, wodurch Bakterienwachstum vollständig unterbunden wird. Das erforderliche Enzym ist allerdings im Handel nicht ohne weiteres in technischen Mengen erhältlich.

Es wurde nun gefunden, daß trägergebundene Pilzlactasen, die ein Wirkungsoptimum im sauren Bereich haben und bei diesem Optimum hitzeempfindlich sind, überraschenderweise außerhalb des Wirkungsoptimums im neutralen Bereich eine viel höhere Hitzebeständigkeit aufweisen, so daß sie bei Temperaturen oberhalb 50°C einsetzbar sind. Während die aus Asp. oryzae gewonnene Pilzlactase bei pH 4,5 und 55°C nur eine Halbwertzeit von 7 Tagen hat, beträgt die Halbwertzeit bei der gleichen Temperatur und pH 6,5 etwa 2 Monate.

Gegenstand der Erfindung ist das in den Patentansprüchen näher gekennzeichnete Verfahren. Für die technische Durchführung des neuen Verfahrens kommt vor allem die im Handel zu günstigen Preisen leicht erhältliche Lactase aus Aspergillus oryzae-Kulturen in Betracht. Die als Nebenaktivität auftretenden Proteasen sind weniger hitzebeständig und werden bei der Hydrolysetemperatur inaktiviert. Dadurch wird die durch Proteasen ausgelöste Freisetzung von Bitterstoffen unterdrückt. Auf die hohe Hydrolysetemperatur ist auch die hohe, für ein technisches Verfahren ausreichende Spaltungsgeschwindigkeit zurückzuführen, was außerhalb des Wirkungsoptimums nicht zu erwarten war.

Pilzlactasen werden im allgemeinen durch die Hydrolyseprodukte, Glucose und Galaktose, gehemmt. Um den Einfluß dieser Hemmung gering zu halten, wird das Verfahren in einem Säulenreaktor mit geringer Rückvermischung durchgeführt.

1159078

Die Lactase kann covalent, adsorptiv oder durch Einschluß an einen geeigneten Träger gebunden sein. Eine gute Durchströmung des Säulenreaktors wird mit perlförmigen Trägerpartikeln mit Teilchengrößen über 20 µm erreicht. Auch in Fasern oder Folien eingeschlossene Enzyme sind verwendbar. Insbesondere für die Verarbeitung von Milch hat sich der Einsatz eines Trägermaterials mit einem Ausschluß-Molekulargewicht bis 1 Million (vgl. W. Heitz, Angew. Chemie, Bd. 82, 1970, S. 675 - 689) als vorteilhaft erwiesen. Stoffe mit höherem Molekulargewicht dringen nicht in den Träger ein und werden ungehindert eluiert. Sie verstopfen dadurch nicht das Trägerinnere mit seinem Aktivitätspotential. Das gleiche gilt für Fettemulsionströpfchen der Milch.

Vorzugsweise wird eine covalent an den Träger gebundene Lactase eingesetzt. Zur Aufrechterhaltung des neutralen Milieus im Reaktor werden carboxylgruppenfreie Träger bevorzugt. Hierzu gehören vor allem Oxirangruppen enthaltende Trägerperlen auf Basis von Acryl- oder insbesondere Methacrylamid. Solche Träger sind z.B. in den DE-AS 2 237 316 und 2 343 633 sowie DE-OS 27 22 751 beschrieben.

Die Bindung des Enzyms erfolgt in üblicher Weise. Die Aktivität des immobilisierten Enzyms liegt im allgemeinen im Bereich von 100 bis 10.000 U/g Feuchtgewicht, und vorzugsweise zwischen 500 und 2000 U/g. Zur technischen Durchführung des Verfahrens wird der beladene Enzymträger in ein senkrecht stehendes, thermostatisiertes Rohr gefüllt und dieses kontinuierlich durchströmt.

Als Substratlösungen kommen wäßrige Lactoselösungen mit einem Gehalt von 1 bis 250 g Milchzucker/l in Frage. Der pH-Wert kann zwischen 6 und 7 liegen. Das bevorzugte Substrat ist Milch in der Form von Voll- oder Magermilch. Außerdem können Süßmolke oder neutrales Permeat mit Vorteil erfindungsgemäß verarbeitet werden. Die Hydrolysetemperatur liegt im Regelfall zwischen 50 und 60°C, bei Milch vorzugsweise etwa bei 55 bis 60°C. Bei der Milchzuckerspaltung in Vollmilch wurde mit einer Pilzlactase aus Asp. oryzae, gebunden an Oxiran-Methacrylamid-Perlen, bei einer Durchflußrate von etwa 20 ml/h·g Feuchtperlen ein Hydrolysegrad von 88 bis 95 % erreicht. Innerhalb von zwei Monaten sank die Aktivität auf die Hälfte. Es wurde kein Bakterienwachstum beobachtet.

Beispiel

A) Immobilisierung der Lactase

500 mg Aspergillus oryzae-Lactase mit 15.000 U/g (1 U ist jene Aktivität, die pro Minute aus einer 0,15 %igen ortho-Nitrophenyl-galactopyranosid-Lösung bei 30°C, pH = 4,5, 1 µMol des Substrates spaltet) wurde über Nacht durch Schütteln in 10 ml 0,9 molarem Phosphatpuffer von pH = 7,5 bei Raumtemperatur an 1 g epoxidfunktionellen, porösen Träger auf Polymethacrylamidbasis (Eupergit C® von Röhm GmbH) gekuppelt. Seine GPC-Ausschlußgrenze liegt für Dextrane bei einem mittleren Molekulargewicht von 300.000; der mittlere Perldurchmesser ist 80 µm. Man erhält 3,1 g Feuchtträger mit einer Aktivitätsausbeute von 47 % (910 U/g Feuchtträger).

B) Reaktorversuche

1. Vollmilch

3 g Immobilisat werden in ein auf 56°C thermostatisiertes doppelwandiges Rohr von 0,9 cm Innendurchmesser und 10 cm Länge eingefüllt. Beide Enden werden mit Nylongaze von 400 mesh verschlossen. Sterile Vollmilch (3,5 % Fett) wird mit einer Durchflußrate von 60 ml/h kontinuierlich von oben nach unten durch den Reaktor geschickt. Der Hydrolysegrad zu Beginn des Versuches liegt bei 92 %. Täglich werden Proben gezogen. Außerdem wird das Hydrolyseverfahren einmal täglich unterbrochen und das Festbett mit 0,1 %iger Essigsäure 30 Minuten bei 40°C gespült; davor und danach wird mit Wasser gewaschen. Nach 30 Tagen Dauerbetrieb ist kein mikrobieller Befall feststellbar. Der Hydrolysegrad liegt immer noch bei 68 %. Daraus läßt sich eine Halbwertzeit der Aktivität von ca. 60 Tagen extrapolieren. Die hydrolysierte Milch ist von angenehm süßem Geschmack ohne jede Spur von Bitterkeit.

2. Süßmolke

Für den Reaktor-Dauerbetrieb wird die Süßmolke mit einer Klärzentrifuge von Trübstoffen befreit. Der Dauerbetrieb zur Hydrolyse der in Süßmolke enthaltenen Lactose erfolgt analog Beispiel 1. Der Hydrolysegrad von anfangs 96 % sinkt innerhalb von 60 Tagen auf 60 %. Daraus ergibt sich eine Halbwertzeit von 3 Monaten. Im Beobachtungszeitraum wurde keine mikrobielle Kontamination in der Säule festgestellt.

Patentansprüche

1. Verfahren zur Spaltung von Milchzucker in wäßriger, etwa neutraler Lösung mittels einer trägergebundenen nicht-bakteriellen Lactase in einem Säulenreaktor,

dadurch gekennzeichnet,

daß man die wäßrige Milchzuckerlösung bei einer Temperatur über 50°C über einen mit einer Pilzlactase beladenen Enzymträger fließen läßt.

2. Verfahren nach Anspruch 1, dadurch gekennzeichnet, daß als Milchzuckerlösung Süßmolke, neutrales Permeat oder insbesondere Milch eingesetzt wird.

3. Verfahren nach den Ansprüchen 1 und 2, dadurch gekennzeichnet, daß es bei einer Temperatur zwischen 50 und 60°C , vorzugsweise 55 - 60°C, durchgeführt wird.

4. Verfahren nach den Ansprüchen 1 bis 3, dadurch gekennzeichnet, daß ein Enzymträger mit einem Ausschluß-Molekulargewicht bis 1 Million eingesetzt wird.

5. Verfahren nach den Ansprüchen 1 bis 4, dadurch gekennzeichnet, daß ein Enzymträger mit covalent gebundener Pilzlactase eingesetzt wird.

6. Verfahren nach den Ansprüchen 1 bis 5, dadurch gekennzeichnet, daß ein Enzymträger mit einer Teilchengröße über 20 µm eingesetzt wird.

7. Verfahren nach den Ansprüchen 1 bis 6, dadurch gekennzeichnet, daß ein mit Pilzlactase von Aspergillus oryzae beladener Enzymträger eingesetzt wird.

8. Trägergebundenes Pilzlactase-Präparat, dadurch gekennzeichnet, daß es Pilzlactase enthält, die covalent an ein Trägermaterial mit einem Ausschluß-Molekulargewicht bis 1 Million gebunden ist.

9. Trägergebundenes Pilzlactase-Präparat nach Anspruch 8, dadurch gekennzeichnet, daß es eine covalent gebundene Lactase aus Aspergillus, insbesondere Aspergillus oryzae, enthält.

Europäisches Patentamt

# EUROPÄISCHER RECHERCHENBERICHT

0061530

Nummer der Anmeldung

EP 81 11 0022

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X,Y | JOURNAL OF DAIRY SCIENCE, Band 63, Mai 1980, Sup. 1, Seite 63, DR66<br>B.A. FRIEND et al.: "Characterization and evaluation of an immobilized lactase system" * Zusammenfassung * | 1-9 | A 23 C 9/12<br>C 12 P 19/14<br>C 12 N 11/08 |
| X,Y | JOURNAL OF MILK FOOD TECHNOLOGY, Band 36, Nr. 1, 1973, Seiten 31-33<br>J.H. WOYCHIK et al.: "Lactose hydrolysis in milk and milk products by bound fungal beta-galactosidase" * Seite 31, Spalte 2 - Seite 32, Spalten 2 und 3 * | 1-6,8, 9 | |
| D,X | DE-A-2 818 086 (SUMITOMO CHEMICAL)<br>* Anspruche 1,18; Seite 33, Versuch 4 * | 1,3,5, 7 | RECHERCHIERTE SACHGEBIETE (Int. Cl. 3) |
| Y | JOURNAL AND FOOD SCIENCE, Band 38, 1973, Seiten 1070-1073<br>L.E. WIERZBICKI et al.: "Immobilization of microbial lactases by covalent attachment to porous glass" * Seite 1070, Spalte 2, Absatz 3 * | 6 | A 23 C 9/00<br>A 23 C 21/00 |

Der vorliegende Recherchenbericht wurde für alle Patentansprüche erstellt.

| Recherchenort | Abschlußdatum der Recherche | Prüfer |
|---|---|---|
| DEN HAAG | 23-06-1982 | DESMEDT G.R.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82